(19) Europäisches Patentamt
European Patent Office
Office européen des brevets

(11) Publication number: **0 444 706 A2**

(12) **EUROPEAN PATENT APPLICATION**

(21) Application number: **91103131.8**

(22) Date of filing: **01.03.91**

(51) Int. Cl.5: **C07D 265/30**, C07D 413/12, C07F 7/08, C07C 217/62, A01N 43/84, A01N 55/00

(30) Priority: **02.03.90 IT 1954090**

(43) Date of publication of application: **04.09.91 Bulletin 91/36**

(84) Designated Contracting States: **AT BE CH DE DK ES FR GB GR LI NL SE**

(71) Applicant: **MINISTERO DELL' UNIVERSITA' E DELLA RICERCA SCIENTIFICA E TECNOLOGICA**
**76, Lungotevere Thaon di Revel**
**I-00196 Roma(IT)**

(72) Inventor: **Camaggi, Giovanni, Dr,**
**Via Ragazzi del 99**
**I-28100 Novara(IT)**
Inventor: **Filippini, Lucio, Dr.**
**27, Via F.lli Cervi**
**I-21047 Saronno Varese(IT)**
Inventor: **Gusmeroli, Marilena, Dr.**
**31, Via della Guerrina**
**I-20052 Monza Milan(IT)**
Inventor: **Garavaglia, Carlo, Dr.**
**7, Piazza Vittoria**
**I-20012 Cuggiono Milan(IT)**
Inventor: **Mirenna, Luigi**
**4, Via Gamboloita**
**I-20139 Milan(IT)**

(74) Representative: **Weinhold, Peter, Dr. et al**
**Patentanwälte Dipl.-Ing. G. Dannenberg Dr. P. Weinhold Dr. D. Gudel Dipl.-Ing. S. Schubert Dr. P. Barz Siegfriedstrasse 8**
**W-8000 München 40(DE)**

# (54) Aryl-propyl-amines having antifungal activity.

(57) Disclosed are compounds endowed with antifungal activity, said compounds having the general formula (I):

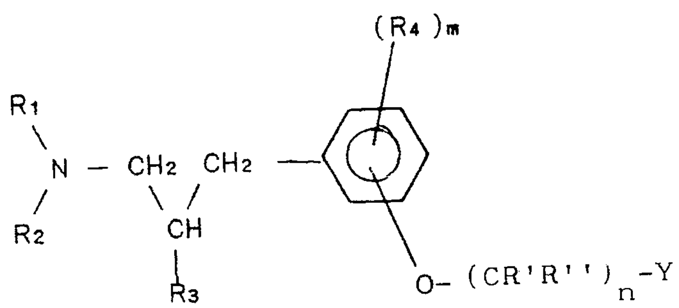

(I)

wherein:

$R_1$ and $R_2$, among others, represent hydrogen, ($C_1$-$C_6$)-alkyl groups and groups Ar-B in which
Ar
is a ($C_6$-$C_{10}$)-(halo)-aryl group and
B
is an (alkyl)-($C_1$-$C_4$)-alkylene group,
$R_3$ is a ($C_1$-$C_3$)-alkyl group;
$R_4$ represents halogen or ($C_1$-$C_3$)-(halo)-alkyl;

EP 0 444 706 A2

| | |
|---|---|
| m | ranges from 0 to 4; |
| R',R", | represent H, $(C_1-C_3)$-alkyl or halogen; |
| n | ranges from 0 to 3; |
| Y | represents an optionally substituted $-CH=CH_2$, $(C_3-C_6)$-cycloalkyl, 5- or 6-membered heterocyclic or silyl group. |

The present invention relates to aryl-propyl-amines endowed with a high antifungal activity, to a process for preparing them and to their use as fungicides in agriculture.

Therefore, one object of the present invention are compounds of general formula (I):

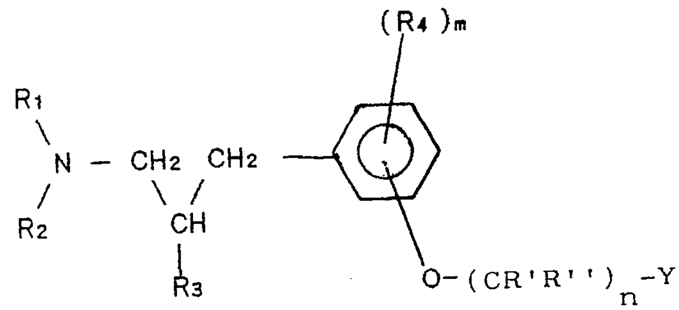

(I)

wherein:

$R_1$ and $R_2$, the same of different from each other, represent hydrogen, linear or branched ($C_1$-$C_6$)-alkyl groups, groups Ar-B in which

A

is a ($C_6$-$C_{10}$)-aryl or ($C_6$-$C_{10}$)-halo-aryl group (e.g. phenyl, naphthyl and the corresponding groups having one or more halogen, particularly chloro, substituents) and

B

is a ($C_1$-$C_4$)-alkylene or ($C_1$-$C_2$)-alkyl-($C_1$-$C_4$)-alkylene group,

or, taken together with each other and with the N atom, represent a ($C_3$-$C_8$)-heterocyclic group or a ($C_2$-$C_7$)-heterocyclic group containing a second heteroatom selected from 0 and S, said heterocyclic groups being optionally substituted, preferably with one or more ($C_1$-$C_4$)-alkyl groups, halogen atoms (e.g. F, Cl, Br, I, particularly Cl), ($C_6$-$C_{10}$)-aryl groups, and groups Ar-B as defined above;

$R_3$ represents a ($C_1$-$C_3$)-alkyl group (e.g. methyl or ethyl);

$R_4$ represents halogen, ($C_1$-$C_3$)-alkyl or ($C_1$-$C_3$)-halo-alkyl groups (e.g. methyl, ethyl and the corresponding groups having one or more halogen, particularly Cl substituents); in case m is $\geqq 2$ the groups $R_4$ being the same or different from each other;

m is an integer of from 0 to 4, particularly 0, 1 or 2;

R', R'', the same or different from each other, represent hydrogen, ($C_1$-$C_3$)-alkyl (e.g. methyl or ethyl) and halogen atoms (particularly F and Cl);

n is an integer of from 0 to 3;

y represents a $-CH=CH_2$ (ethenyl) group, a ($C_3$-$C_6$)-cycloalkyl group (e.g. cyclopentyl and cyclohexyl), a 5- or 6-membered heterocyclic group, said groups being optionally substituted, preferably with one or more groups selected from halogen atoms, ($C_1$-$C_4$)-alkyl groups, (e.g. methyl or ethyl), ($C_1$-$C_2$)-haloalkyl groups (see above), ($C_1$-$C_3$)-alkoxy groups (e.g. methoxy or ethoxy) and ($C_1$-$C_3$)-halo-alkoxy groups; or represents a group of formula

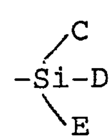

wherein

C, D, E,

the same or different from one another, represent hydrogen, ($C_1$-$C_4$)-alkyl groups, ($C_1$-$C_4$)-haloalkyl groups, ($C_1$-$C_4$) -alkoxy groups, ($c_1$-$C_4$)-haloalkoxy groups, ($C_6$-$C_{10}$)-aryl groups, and ($C_6$-$C_{10}$)-haloaryl groups (for specific examples of said groups see above).

The compounds of general formula (I) contain at least one asymmetric centre; the pure enantiomers or pure diastereoisomers, as well as mixtures thereof in any ratios, are comprised within the scope of the instant invention.

As used herein, "halogen" or "halo" denotes F, Cl, Br and I, if not specified otherwise.

Examples of aryl groups are phenyl, naphthyl and higher homologues.

Preferred examples of groups Ar-B are benzyl and 3-phenyl-propyl. Preferred examples of groups of formula

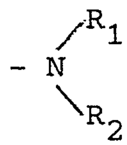

wherein N, $R_1$ and $R_2$ together represent a ($C_3$-$C_8$)- or ($C_2$-$C_7$)-heterocyclic group as defined above, are those groups which are derived from morpholine, 2,6-dimethyl-morpholine, piperidine, 2,6-dimethyl-piperidine and thiomorpholine. Said groups can also be substituted as defined above.

Preferred examples of 5- or 6-membered heterocyclic groups Y are those derived from pyridine, pyrimidine, thiophene, thiazole, oxazole, isoxazole and derivatives thereof which contain a fused benzene ring. Said groups may optionally be substituted as defined above.

Among the groups Y which represent ($C_3$-$C_6$)-cycloalkyl groups, the cyclohexyl, cyclopropyl, cyclopentyl and cyclobutyl groups, optionally substituted as defined above, such as 1-methyl-2,2-dichloro-cyclopropyl, may be mentioned.

Among the silyl groups of formula SiCDE, trimethyl-silyl, tert.-butyl-dimethyl-silyl and dimethyl-phenyl-silyl groups are preferred examples.

Further objects of the present invention are:

- the salts of the compounds of general formula (I) deriving from an inorganic acid such as, e.g., a hydrogen halide (e.g. hydroiodic, hydrobromic or hydrochloric acid); sulfuric acid, nitric acid, thiocyanic acid and phosphoric acid; or from an organic acid such as, e.g., acetic acid, propanoic acid, ethane-dicarboxylic acid, propane-dicarboxylic acid, benzoic acid, salicyclic acid, saccharin, methane-sulfonic acid and 4-methyl-benzene-sulfonic acid;
- the metal complexes obtainable by a complex-forming reaction between the compounds of general formula (I) and an organic or inorganic metal salt such as, e.g., a halide, nitrate, sulfate, phosphate of, e.g., copper, manganese, zinc or iron. Said salts and complexes may be prepared according to well-known techniques.

The compounds of general formula (I) can be prepared by known, i.e., conventional methods.

A preferred method can be schematically represented as follows (for $\underline{n}$ = 1):

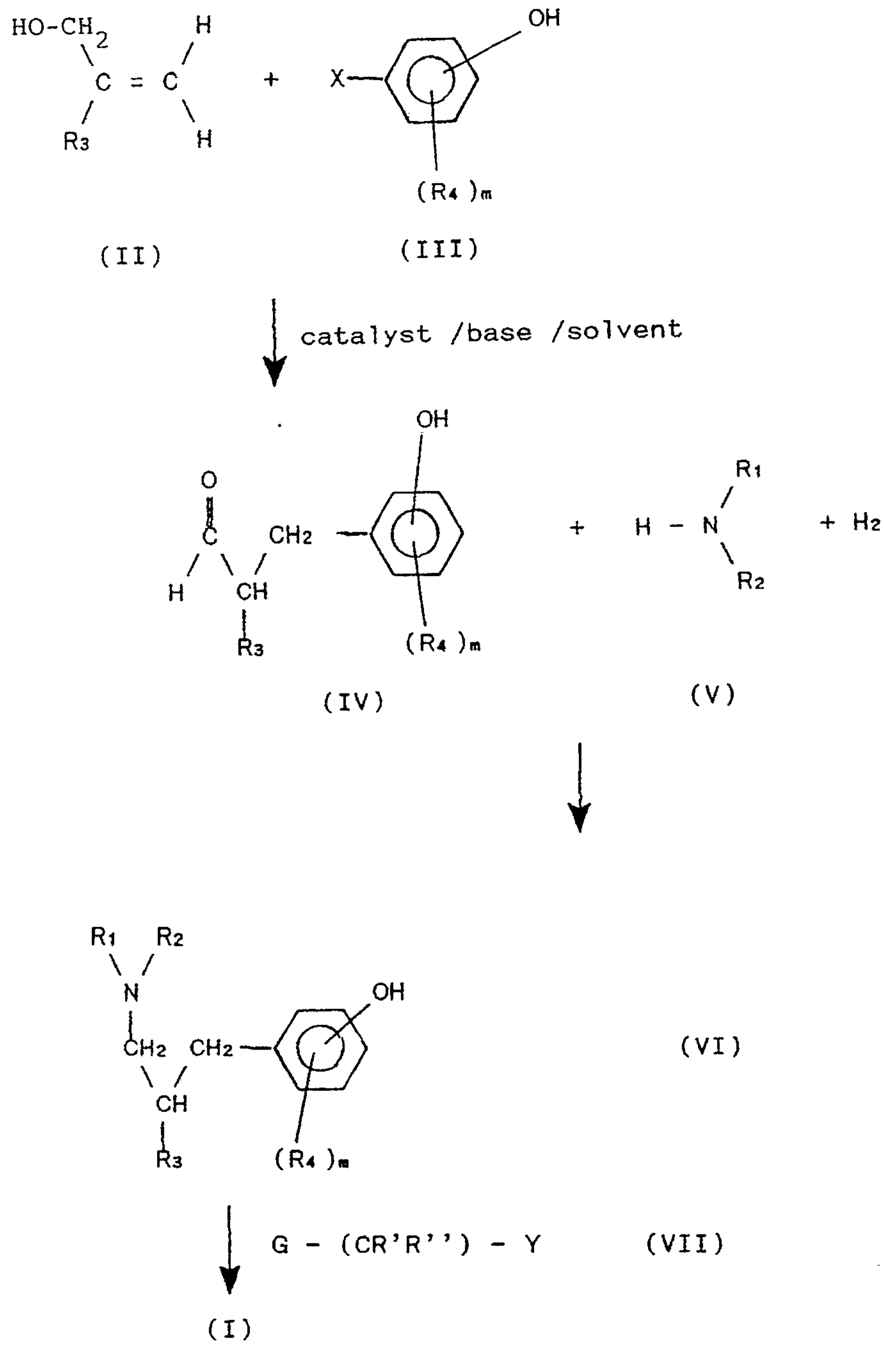


(II) (III) (IV) (V) (VI) (VII) (I)

More specifically, the allyl alcohol (II) is reacted with the phenol (III), wherein X represents halogen (particularly Br or I) or an activated ester group (preferably derived from trifluoromethane-sulfonic ester), in the presence of a Pd-(II) salt [such as Pd-(II) chloride and Pd-(II) acetate] and an organic base (such as triethylamine and tributylamine) or an inorganic base (such as sodium bicarbonate and potassium bicarbonate), in a dipolar protic solvent (such as water and ethanol) or aprotic solvent (such as N,N-dimethylformamide and N-methyl-pyrrolidone), or mixtures of such solvents, at a temperature of from about 0° C to about the boiling temperature of the solution.

The addition of phosphines such as tris-(ortho-tolyl)-phosphine or triphenyl-phosphine may prove advantageous (see JOC 41 1206, 1976).

The thus obtained aldehyde (IV), after the addition of the amine (V), is hydrogenated in situ to yield the amine (VI). The addition of a conventional hydrogenation catalyst (such as Pd supported on charcoal and Raney-nickel) may prove advantageous. The hydrogen pressure usually ranges from about 1 to about 10 atmospheres, and the temperature is usually in the range of from about 0° C to about 60° C (see J. March "Advanced Organic Chemistry", 2nd Edition, Int. St. Edition, page 819).

In the above preparation scheme the symbols $R_1$, $R_2$, $R_3$, $R_4$, R', R'', Y and m have the meanings

defined above.

By reacting the amine (VI) with the compound (VII), compound (I) is obtained. In compound (VII), G stands for halogen (preferably Cl, Br or I) or an activated ester group (preferably methane-sulfonic ester or p-toluene-sulfonic ester). The reaction is carried out in the presence of an organic base (such as triethylamine and pyridine) or an inorganic base (such as sodium carbonate and sodium bicarbonate) in a dipolar protic solvent (such as water and ethanol) or aprotic solvent (such as N,N-dimethyl-formamide and N-methyl-pyrrolidone) at temperatures of from about 25° C to the boiling temperature of the solution (see J. March "Advanced Organic Chemistry", 2nd Edition, Int. St. Edition, page 357).

If desired, the metal salts and/or complexes of compounds (I) can then be prepared according to well-known techniques.

The alcohols (II) and phenols (III) are commercially available or can be prepared according to known techniques.

The compounds (VII) are also commercially available. When Y represents the group

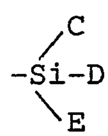

and at least one of the radicals C, D and E is ($C_1$-$C_4$)-perfluoroalkyl, compound (VII) can be obtained according to methods known from the literature [JACS 73, 3518 (1951), Tetrahedron Letters 25, 2195 (1984)].

The amines (V) are products available on the market or they can easily be synthesized (see J. March "Advanced Organic Chemistry", 2nd Edition, Int. St. Edition, page 357).

The compounds of general formula (I) are endowed with a high activity as growth inhibitors of several species of pathogenic fungi which attack the cultivations of useful plants.

When they are applied to useful plants or parts thereof such as, e.g., leaves, the compounds of formula (I) show both a preventive and a curative activity, and are particularly effective in preventing diseases caused by pathogenic fungi, such as, e.g., those belonging to the genera Erysiphe and Helminthosporium.

Examples of plant diseases which can be controlled by the compounds according to the present invention are:

- Erysiphe graminis on cereals,
- Sphaeroteca fuliginea on Cucurbitaceae (e.g., cucumber),
- Puccinia on cereals,
- Septoria on cereals,
- Helminthosporium on cereals,
- Rhynchosporium on cereals,
- Podosphaera leucotricha on apple-trees,
- Uncinula necator on vines,
- Venturia inaequalis on apple-trees,
- Pyricularia oryzae on rice,
- Botrytis cinerea,
- Fusarium on cereals,

as well as other diseases.

For practical use in agriculture, it is often useful to have available antifungal compositions containing one or more components of formula (I) as active substances.

The application of these compositions may take place on any part of the plant, such as, e.g., leaves, stems, branches and roots, or on the seeds, before sowing, or also on the soil in which the plant grows. The compositions can be used in the form of dry powders, wettable powders, emulsifiable concentrates, pastes, granulates, solutions, suspensions, and the like; the selection of the particular type of composition will depend on the specific use.

The above compositions may be prepared in known manner, for example by diluting or dissolving the active substance with/in a solvent medium and/or a solid diluent, optionally in the presence of surfactants. As solid diluents, or supports, the following may, for instance, be used: silica, kaolin, bentonite, talc, fossil meal, dolomite, calcium carbonate, magnesium oxide, gypsum, clays, synthetic silicates, attapulgite and sepiolite.

The liquid diluents may be selected from water and various types of (organic) solvents such as, e.g.,

aromatic solvents (such as benzene, xylenes and mixtures of alkyl-benzenes), chloroaromatic compounds (such as chlorobenzene), paraffins (such as petroleum cuts), alcohols (such as methanol, propanol and butanol), amines, amides (e.g. dimethylformamide), ketones (e.g. cyclohexanone, acetophenone, isophorone and ethyl-amylketone) and esters (e.g. isobutyl acetate).

As surfactants, the following may, for instance, be used: sodium, calcium or triethanolamine salts of alkyl-sulfates, alkyl-sulfonates, alkyl-aryl-sulfonates, polyethoxylated alkylphenols, adducts of ethylene oxide with fatty alcohols, polyethoxylated fatty acids, polyethoxylated sorbitol esters, polyethoxylated fats and ligno-sulfonates.

The compositions may also contain special additives for particular purposes, such as, e.g., binders such as gum arabic, polyvinyl alcohol and polyvinylpyrrolidone.

If desired, also other active substances such as fungicides, plant growth regulators, herbicides, insecticides, fertilizers etc. can be added to the compositions according to the present invention.

The concentration of the active substance of formula (I) in the above compositions may vary as a function of the active compound, of the culture, of the pathogen, of environmental conditions and of the type of formulation adopted. Generally, however, the concentration of active agent will range from about 0.1% to about 95%, and preferably from about 0.5% to about 90% by weight.

The following examples are to illustrate the present invention without being a limitation thereof.

## EXAMPLE 1

(a) Synthesis of 4-{3-[4-(trimethylsilylmethoxy)-phenyl]-2-methylpropyl}-2,6-dimethyl-morpholine (Compound No. 1)

1 g of 4-[3-(4-(hydroxy-phenyl)-2-methylpropyl]-2,6-dimethyl-morpholine is dissolved in 10 ml of dimethylformamide. To the resulting solution 0.7 g of anhydrous sodium carbonate are added, the resulting mixture is heated to 80 °C under a nitrogen blanketing atmosphere, and is kept heated at this temperature for 30 minutes. Then 0.6 g of potassium iodide and 0.65 g of chloromethyl-trimethyl-silane are added and the heating of the mixture is continued for a further 4 hours. The reaction mixture is poured into water and then extracted with ethyl ether. The etheral extract is then thoroughly dried and evaporated under reduced pressure. The resulting crude product is purified by chromatography on silica gel, with hexane/ethyl acetate = 9:1 as the eluent. Thus 0.5 g of compound 1 are obtained.

Analysis:

NMR (60 MHz) in $CDCl_3$:

$$\delta = 6.7 \quad (4H, m)$$
$$= 3.5 \quad (4H, m)$$
$$= 0.8 - 2.6 \quad (18H, m)$$
$$= 0.0 \quad (9H, s)$$

(b) Synthesis of 4-[3-(4-hydroxy-phenyl)-2-methylpropyl]-2,6-dimethyl-morpholine used as starting material

1.1 g of p-iodo-phenol is added to a solution of beta-methallyl alcohol (0.6 g) in 20 ml of deionized water and 2.5 ml of N-methyl-pyrrolidone. The solution is purged with nitrogen, and then potassium carbonate (2.07 g) and palladium acetate (0.011 g) are added. The resulting mixture is heated to 80 °C and is kept at this temperature for 10 hours. Then 2,6-dimethyl-morpholine (1.8 g) is added and the atmosphere inside the reaction vessel is replaced by hydrogen (1.5 atm), with the reaction mixture being kept vigorously stirred at a temperature of 40 °C. When the reaction is complete, the reaction mixture is extracted with methylene chloride, the extract is thoroughly dried and is evaporated under reduced pressure.

The resulting crude product is purified by chromatography on silica gel, with hexane/ethyl acetate = 3:2 as the eluent. Thus 0.9 g of desired product are obtained.

Analysis:

NMR (60 MHz) in $CDCl_3$:

$\delta$ = 6.7 (4H, m)
= 5.1 (1H, s)
= 3.5 (2H, m)
= 0.8 - 2.6 (18H, m)

EXAMPLE 2

In similar manner, employing the corresponding starting materials, the following compounds 2 to 11 where synthesized.

Compound No. 2

4-{3-[3-(2-(4,6-Dichloro)-pyridyloxy)-phenyl]-2-methylpropyl}-2,6-dimethyl-morpholine.
NMR (60 MHz) in $CDCl_3$:

$\delta$ = 7.9 (1H, d)
= 7.7 (1H, d)
= 6.9 (4H, m)
= 3.6 (2H, m)
= 0.8 - 2.8 (18H, m)

Compound No. 3

4-{3-[3-(2-(4-Nitro)-pyridyloxy)-phenyl]-2-methylpropyl}-2,6-dimethyl-morpholine.
NMR (60 MHz) in $CDCl_3$:

$\delta$ = 8.9 (1H, d)
= 8.3 (1H, dd)
= 6.9 - 7.0 (5H, m)
= 3.5 (2H, m)
= 0.8 - 2.8 (18H, m)

Compound No. 4

4-{3-[3-(Cyclopentyloxy)-phenyl]-2-methylpropyl}-2,6-dimethyl-morpholine.
NMR (60 MHz) in $CDCl_3$:

$\delta$ = 7.1 (1H, t)
= 6.7 (3H, m)
= 4.7 (1H, m)
= 3.6 (2H, m)
= 0.8 - 3.0 (26H, m)

Compound No. 5

4-{3-[3-(Cyclopropylmethoxy)-phenyl]-2-methylpropyl)-2,6-dimethyl-morpholine.
NMR (60 MHz) in $CDCl_3$:

δ = 6.5 - 7.2 (4H, m)
= 3.6 (4H, m)
= 0.2 - 2.8 (23H, m)

Compound No. 6

4-{3-[3-(Trimethylsilylmethoxy)-phenyl]-2-methylpropyl}-2,6-dimethyl-morpholine.
NMR (60 MHz) in $CDCl_3$:

δ = 6.3 - 7.0 (4H, m)
= 3.6 (4H, m+s)
= 0.8 - 2.9 (18H, m)
= 0.0 (9H, s)

Compound No. 7

4-{3-[4-(2-(4,6-(Dichloro)-pyridyloxy)-phenyl]-2-methylpropyl}-2,6-dimethyl-morpholine.
NMR (60 MHz) in $CDCl_3$:

δ = 7.9 (1H, d)
= 7.7 (1H, d)
= 7.0 (4H, m)
= 3.6 (2H, m)
= 0.8 - 2.9 (18H, m)

Compound No. 8

4-{3-[4-(2-Pyrimidyloxy)-phenyl]2-methylpropyl}-2,6-dimethyl-morpholine.
NMR (60 MHz) in $CDCl_3$:

δ = 8.5 (2H, d)
= 7.0 (5H, m)
= 3.6 (2H, m)
= 0.8 - 2.8 (18H, m)

Compound No. 9

4-{3-[4-(Cyclopropyl-methoxy)-phenyl]-2-methylpropyl}-2,6-dimethyl-morpholine.
NMR (60 MHz) in $CDCl_3$:

$$\delta = 6.9 \quad (4H, m)$$
$$= 4.7 \quad (1H, m)$$
$$= 3.6 \quad (2H, m)$$
$$= 0.8 - 2.8 \quad (26H, m)$$

Compound No. 10

4-{3-[3-(Cyclohexyl-methoxy)-phenyl]-2-methylpropyl}-2,6-dimethyl-morpholine.
NMR (60 MHz) in $CDCl_3$:

$$\delta = 6.4 - 7.1 \quad (4H, m)$$
$$= 3.6 \quad (4H, m)$$
$$= 0.6 - 2.8 \quad (29H, m)$$

Compound No. 11

4-{3-[4-(tert.-Butyl-dimethyl-silyl-oxy)-phenyl]-2-methylpropyl}-2,6-dimethyl-morpholine.
NMR (60 MHz) in $CDCl_3$:

$$\delta = 6.8 \quad (4H, m)$$
$$= 3.6 \quad (4H, m+s)$$
$$= 0.8 - 2.8 \quad (27H, m)$$
$$= 0.0 \quad (6H, s)$$

EXAMPLE 3

Determination of the preventive fungicidal activity towards Helminthosporium teres

Both faces of leaves of plants of barley cv. Arna, grown in pots in a conditioned atmosphere, were sprayed with the tested products (Compounds Nos. 5 and 10) in water-acetone solution at 20% of acetone (volume/volume).

After 2 days in an atmosphere conditioned at 20°C and 70% R.H., both faces of the leaves of the plants were sprayed with an aqueous suspension of Helminthosporium teres (250,000 conidia/ml). After a further 24 hours in an atmosphere saturated with humidity, at 21°C, the plants were kept in a conditioned environment for fungus incubation.

At the end of said period (12 days), the severity of the infection was examined visually and rated according to a scale ranging from 100 (healthy plant) to 0 (completely infected plant).

The data obtained is summarized in Table 1.

TABLE 1

| COMPOUND NO. | DOSE (ppm) | HELMINTHOSPORIUM RATING |
|---|---|---|
| 5 | 500 | 100 |
| | 125 | 100 |
| 10 | 500 | 100 |
| | 125 | 100 |

EXAMPLE 4

Determination of the fungicidal activity towards corn oidium (Erysiphe graminis D.C.)

Preventive Activity:

Both faces of leaves of plants of corn cv. Irnerio, grown in pots in a conditioned environment, were sprayed with the tested products (Compounds Nos. 1 and 9) in water-acetone solution at 20% of acetone (volume/volume).

After 1 day in an atmosphere conditioned at 20 °C and 70% R.H., both faces of the leaves of the plants were sprayed with an aqueous suspension of Erysiphe graminis (200,000 conidia/ml). After a further 24 hours in an atmosphere saturated with humidity, at 21 °C, the plants were kept in a conditioned atmosphere for fungus incubation.

At the end of said incubation period (12 days), the severity of the infection was examined visually and rated according to a scale ranging from 100 (healthy plant) down to 0 (completely infected plant).

Curative Activity:

Both faces of leaves of plants of corn cv. Irnerio, grown in pots in a conditioned atmosphere, were sprayed with an aqueous suspension of Erysiphe graminis (200,000 conidia/ml). After 24 hours in an atmosphere saturated with humidity, at 21 °C, the leaves were sprayed with the tested products (Compounds Nos. 1 and 9) in water-acetone solution at 20% of acetone (volume/ volume).

At the end of the fungus incubation period (12 days), the severity of the infection was examined visually and rated according to a scale ranging from 100 (healthy plant) down to 0 (completely infected plant).

The data obtained is summarized in Table 2.

TABLE 2

| COMPOUND NO. | DOSE (ppm) | ERYSIPHE RATING |
|---|---|---|
| 1 | 500 | 100 |
| | 250 | 100 |
| | 125 | 99 |
| 9 | 500 | 100 |
| | 250 | 100 |
| | 125 | 100 |

**Claims**

**1.** Compounds of general formula (I):

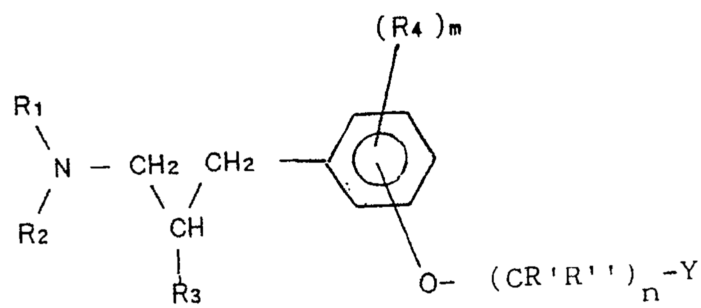

(I)

wherein:

$R_1$ and $R_2$, the same or different from each other, represent hydrogen, linear or branched ($C_1$-

$C_6$)-alkyl groups, groups Ar-B in which
Ar
is a ($C_6$-$C_{10}$)-aryl or ($C_6$-$C_{10}$)-halo-aryl group and
B
is a ($C_1$-$C_4$)-alkylene or ($C_1$-$C_2$)-alkyl-($C_1$-$C_4$)-alkylene group,
or, together with the N atom to which they are bound, represent a ($C_3$-$C_8$)-heterocyclic group or a ($C_2$-$C_7$)-heterocyclic group containing a second heteroatom selected from 0 and S, said heterocyclic groups being optionally substituted;

$R_3$ represents a ($C_1$-$C_3$)-alkyl group;
$R_4$ represents halogen, ($C_1$-$C_3$)-alkyl or ($C_1$-$C_3$)-halo-alkyl;
m is an integer of from 0 to 4;
R',R", the same or different from each other, represent hydrogen, ($C_1$-$C_3$)-alkyl or halogen;
n is an integer of from 0 to 3;
Y represents an optionally substituted ethenyl, ($C_3$-$C_6$)-cycloalkyl or 5- or 6-membered heterocyclic group; or represents a group of formula

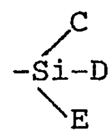

wherein
C, D, E,
the same or different from one another, represent hydrogen, ($C_1$-$C_4$)-alkyl groups, ($C_1$-$C_4$)-haloalkyl groups, ($C_1$-$C_4$)-alkoxy groups, ($C_1$-$C_4$)-haloalkoxy groups, ($C_6$-$C_{10}$)-aryl groups or ($C_6$-$C_{10}$)-haloaryl groups;

as well as enantiomers, diastereoisomers, metal salts and complexes thereof.

2. Compounds according to claim 1, in which said ($C_3$-$C_8$)-or ($C_2$-$C_7$)-heterocyclic group $NR_1R_2$ is selected from morpholinyl, 2,6-dimethyl-morpholinyl, piperidinyl, 2,6-dimethyl-piperidinyl and thiomorpholinyl, said groups being optionally (further) substituted.

3. Compounds according to any one of the preceding claims, in which said ($C_3$-$C_8$)- or ($C_2$-$C_7$)-heterocyclic group $NR_1R_2$ is substituted with at least one group selected from ($C_1$-$C_4$)-alkyl, ($C_6$-$C_{10}$)-aryl, halogen and groups Ar-B in which Ar and B are as defined above.

4. Compounds according to any one of the preceding claims, in which the group Ar-B is selected from benzyl and 3-phenylpropyl.

5. Compounds according to any one of claims 1 to 4, in which Y is selected from pyridinyl, pyrimidinyl, thienyl, thiazolyl, oxazolyl, isoxazolyl, derivatives of said groups which contain a fused benzene ring, cyclohexyl, cyclopropyl, cyclopentyl, cyclobutyl, 1-methyl-2,2-dichloro-cyclopropyl, trimethylsilyl, tert.-butyl-dimethyl-silyl and dimethyl-phenylsilyl.

6. Compounds according to any one of claims 1 to 4, in which Y is selected from ethenyl, ($C_3$-$C_6$)-cycloalkyl and heterocyclic groups, said groups being substituted with at least one group selected from ($C_1$-$C_4$)-alkyl, ($C_1$-$C_2$)-haloalkenyl, ($C_1$-$C_3$)-alkoxy, ($C_1$-$C_3$)-halo-alkoxy and halogen.

7. Compound according to claim 1, i.e., 4-{3-[4-(trimethyl-silylmethoxy)-phenyl]-2-methylpropyl}-2,6-dimethyl-morpholine; 4-{3-[3-(2-(4,6-dichloro)-pyridyloxy)-phenyl]-2-methylpropyl}-2,6-dimethyl-morpholine; 4-{3-[3-(2-(4-nitro)-pyridyloxy)-phenyl]-2-methylpropyl}-2,6-dimethyl-morpholine; 4-{3-[3-(cyclopentyloxy)-phenyl]-2-methyl-propyl}-2,6-dimethyl-morpholine; 4-{3-[3-(cyclopropyl-methoxy)-phenyl]-2-methylpropyl}-2,6-dimethyl-morpholine; 4-{3-[3-(trimethylsilylmethoxy)-phenyl]-2-methylpropyl}-2,6-dimethyl-morpholine; 4-{3-[4-(2-(4,6-dichloro)-pyridyloxy)-phenyl]-2-methylpropyl}-2,6-dimethyl-morpholine; 4-{3-[4-(2-pyrimidyloxy)-phenyl]-2-methylpropyl}-2,6-dimethyl-morpholine; 4-{3-[4-(cyclopropyl-methoxy)-phenyl]-2-methylpropyl]-2,6-dimethyl-morpholine; 4-{3-[3-(cyclohexyl-methoxy)-phenyl]-2-methylpropyl}-2,6-dimethyl-morpholine; or 4-{3-[4-(tert.-butyl-dimethyl-silyloxy)-phenyl]-2-methylpropyl}-2,6-dimethyl-morpholine.

8. Process for preparing the compounds defined in any one of the preceding claims comprising the reaction of an allyl alcohol of general formula (II)

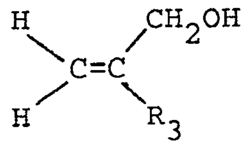

(II)

wherein $R_3$ is as defined in claim 1; with a phenol of general formula (III):

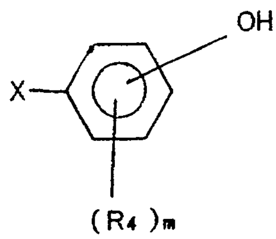

(III)

wherein $R_4$ and m are as defined in claim 1 and wherein X = Br, I or a trifluoromethane-sulfonic ester group, in the presence of Pd(II) and a base, in a dipolar, protic or aprotic solvent, at a temperature of from about 0°C up to the boiling point of the reaction mixture, optionally in the presence of phosphines, to form the aldehyde of general formula (IV):

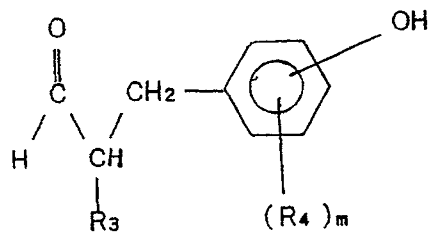

(IV)

which, after the addition of an amine of general formula (V):

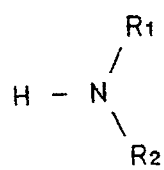

(V)

wherein $R_1$ and $R_2$ are as defined in claim 1; is hydrogenated in situ, optionally in the presence of a hydrogenation catalyst, at a temperature of from about 0 to about 60°C and under an $H_2$ pressure of from about 1 to about 10 atmospheres to form the amine of general formula (VI):

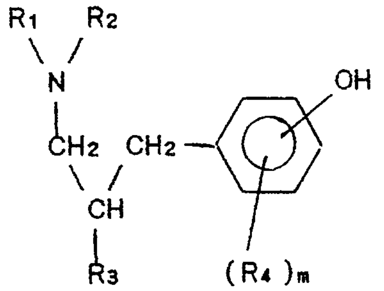

(VI)

which, after reaction with the compound of general formula (VII):

G - $(CR'R'')_n$-Y (VII)

wherein n, R', R'' and Y are as defined in claim 1 and G = Cl, Br, I, a methanesulfonic ester or p-toluene sulfonic ester group; in the presence of a dipolar protic or aprotic solvent at a temperature of from about 25°C to the boiling point of the reaction mixture, yields the compound of general formula (I); and, optionally, the conversion of the obtained compound of general formula (I) into its corresponding salt or metal complex by conventional methods.

**9.** Fungicidal compositions, containing at least one of the compounds according to any one of claims 1 to 7 along with a solvent and/or diluent.

**10.** Use of the compounds according to any one of claims 1 to 7, as agents for inhibiting the growth of pathogenic fungi in cultivations of useful plants, in particular of fungi belonging to the genera Erysiphe and Helminthosporium.